(19) Europäisches Patentamt
European Patent Office
Office européen des brevets

(11) EP 1 797 883 A2

(12) EUROPEAN PATENT APPLICATION

(43) Date of publication:
**20.06.2007 Bulletin 2007/25**

(21) Application number: **07104359.0**

(22) Date of filing: **27.04.2004**

(51) Int Cl.:
***A61K 31/519*** *(2006.01)* ***A61K 31/4468*** *(2006.01)*
***A61K 31/445*** *(2006.01)* ***A61K 31/485*** *(2006.01)*
***A61K 31/454*** *(2006.01)* ***A61K 31/416*** *(2006.01)*
***A61K 31/5377*** *(2006.01)* ***A61K 31/135*** *(2006.01)*
***A61K 31/136*** *(2006.01)* ***A61K 31/4535*** *(2006.01)*
***A61P 29/00*** *(2006.01)*

(84) Designated Contracting States:
**AT BE BG CH CY CZ DE DK EE ES FI FR GB GR HU IE IT LI LU MC NL PL PT RO SE SI SK TR**

(30) Priority: **28.04.2003 US 466116 P**

(62) Document number(s) of the earlier application(s) in accordance with Art. 76 EPC:
**04729640.5 / 1 620 106**

(71) Applicants:
- **Novartis AG**
  **4056 Basel (CH)**
  Designated Contracting States:
  **BE BG CH CY CZ DE DK EE ES FI FR GB GR HU IE IT LI LU MC NL PL PT RO SE SI SK TR**
- **Novartis Pharma GmbH**
  **1230 Vienna (AT)**
  Designated Contracting States:
  **AT**

(72) Inventors:
- **Ganju, Pamposh**
  **London, WC1E 6BS (GB)**
- **Malcangio, Marzia**
  **London, WC1E 6BS (GB)**
- **Song, Chuanzheng**
  **Southborough, MA 01772 (US)**

(74) Representative: **Vögeli-Lange, Regina**
**Novartis AG**
**Corporate Intellectual Property**
**Basel, 4002 (CH)**

Remarks:
This application was filed on 16 - 03 - 2007 as a divisional application to the application mentioned under INID code 62.

(54) **Pharmaceutical composition comprising a cathepsin S inhibitor and an opioid**

(57) The present invention relates to combinations suitable for the treatment of pain of various genesis or aetiology comprising a cathepsin S inhibitor and an opioid, for simultaneous, separate or sequent ial use, to a method of treating or ameliorating pain in a warm-blooded animal in need thereof, to the use of a combination as specified above for the preparation of a medicament for the treatment of pain and to pharmaceutical compositions and commercial packages comprising such combinations.

EP 1 797 883 A2

## Description

The present invention relates to combinations suitable for the treatment of pain of various genesis or aetiology.

The endogenous opi oid system is a major inhibitory system in the central nervous system and plays a pivotal role in the modulation of pain. Activation of opioid receptors (μ-κ and δ) results in analgesia and anti-hyperalgesia in experimental models and in the clinic. The use of opioids is affected by a number of known side-effects and disadvantages such as a decrease in attention and concentration due to sedation, constipation and respiratory depression after taking the drug as well as the risk of drug abuse and drug addiction.

Cathepsin S is a member of the family of lysosomal cysteine cathepsin enzymes, e.g. cathepsins B, K, L and S, which are implicated in various disorders including inflammation, rheumatoid arthritis, osteoarthritis, osteoporosis, tumors (especially tumor invasion and tumor metastasis), coronary disease, atherosclerosis (including atherosclerotic plaque rupture and destabi lization), autoimmune diseases, respiratory diseases, infectious diseases and immunologically mediated diseases (including transplant rejection). As disclosed in WO 03/020287, catheps in S is a suitable target for the development of new therapeutics to treat or ameliorate chronic pain and, hence, a modulator, in particular an inhibitor, of cathepsin S activity can be used to treat or ameliorate chronic pain. Furthermore, WO 03/020287 identifies a number of cathepsin S inhibitors suitable for the treatment of chronic pain. Cathepsin S inhibitors reverse, e.g., mechanical hyperalgesia models of neuropathic pain in rats.

Surprisingly, it has been found that the effect of a combination which comprises a cathepsin S inhibitor and an opi oid is greater than the additive effect of the two single drugs.

Hence, the invention relates to a combination, such as a combined preparation or pharm a-ceutical composition, which comprises a cathepsin S inhibitor, and an opioid, in which the active ingredients are present in each case in free form or in the form of a pharmaceutically acceptable salt and optionally at least one pharmaceutically acceptable carrier; for simultaneous, separate or sequential use.

The term "pain of various genesis or aetiology" includes, but is not limited to, inflammatory pain, hyperalgesia and, in particular, chronic pain, and means in particular pain consequential to trauma, e.g. associated with burns, sprains, fracture or the like, subsequent to surgical intervention, e.g. as post-operative analgesics, chemotherapy-induced pain, as well as inflammatory pain of diverse genesis, e.g. bone and joint pain (osteoarthritis), myofascial pain (muscular injury, fibromyalgia), lower back pain, chronic inflammatory pain, chronic neuropathic pain, e.g. diabetic neuropathy, phantom limb pain and perioperative pain (general surgery, gynecologic surgery) as well as pain associated with, e.g., angina, menstruation or cancer.

The term "opioid" as used herein refers to all drugs, both natural and synthetic, with morphine-like actions. An opioid suitable for the present invention is especially selected from the group comprising alfentanil, allylprodine, alphaprodine, anileridine, benzylmorphine, bezitramide, buprenorphine, butorphanol, clonitazene, codeine, cyclorphan, desomorphine, dextromoramide, dezocine, diampromide, dihydrocodeine, dihydromorphine, eptazocine, ethylmorphine, fentanyl, hydrocodone, hydromorphone, hydroxypethidine, levophenacylmorphan, levorphanol, lofentanil, methylmorphine, morphine, necomorphine, normethadone, normorphine, opium, oxycodone, oxymorphone, pholcodine, profadol and sufentanil.

For instance, alfentanil can be administered, e.g., in the form as marketed, e.g. under the trademark Rapifen™; allylprodine can be administered, e.g., in the form as marketed, e.g. under the trademark Alperidine™; anileridine can be administered, e.g., in the form as marketed, e.g. under the tradem ark Leritine™; benzylmorphine can be administered, e.g., in the form as marketed, e.g. under the trademark Peronine™; bezitramide can be administered, e.g., in the form as marketed, e.g. under the tradem ark Burgodin™; buprenorphine can be administered, e.g., in the form as marketed, e.g. under the trademark Buprenex™; butorphanol can be administered, e.g., in the form as marketed, e.g. under the trademark Torate™; dextromoramide can be administered, e.g., in the form as marketed, e.g. under the tradem ark Palfium™; dezocine can be administered, e.g., in the form as marketed, e.g. under the tradem ark Dalgan™; dihydrocodeine can be administered, e.g., in the form as marketed, e.g. under the tradem ark Novicodin™; dihydromorphine can be administered, e.g., in the form as marketed, e.g. under the trademark Paramorphan™; eptazocine can be administered, e.g., in the form as marketed, e.g. under the tradem ark Sedapain™; ethylmorphine can be administered, e.g., in the form as marketed, e.g. under the tradem ark Dionin™; fentanyl can be administered, e.g., in the form as marketed, e.g. under the trademark Fentanest™ or Leptanal ™; hydrocodone can be administered, e.g., in the form as marketed, e.g. under the trademark Bekadid™ or Calmodid™; hydromorphone can be administered, e.g., in the form as marketed, e.g. under the tradem ark Novolaudon™; hydroxypethidine can be adm inistered, e.g., in the form as marketed, e.g. under the trademark Bemidone™; levorphanol can be administered, e.g., in the form as marketed, e.g. under the trademark Dromoran™; normethadone can be administered, e.g., in the form as marketed, e.g. under the tradem ark Ticarda™; oxycodone can be administered, e.g., in the form as marketed, e.g. under the tradem ark Dihydrone™ and oxymorphone can be administered, e.g., in the form as marketed, e.g. under the tradem ark Numorphan™.

The term "cathepsin S inhibitor" as used herein relates to modulators inhibiting the enzyme activity of cathepsin S and includes, but is not limited to, such modulators as identified in WO03/020287, in particular cathepsin inhibitors,

including cathepsin S specific inhibitors, namely dipeptide nitriles described in published patent application WO99/24460; α-amino fluoro ketones, such as described in US Patent 4,518,528; pept ides with fluoride free leaving groups as described in US Patent 5,374,623; compounds with heterocyclic leaving groups as described in US Patent 5,486,623; {5-(2-morpholinoethoxy)benzofuran-2-carboxylic acid N-(S)-3-methyl-1-{(S)-3-oxo-1-[2-(3-pyridin-2-ylphenyl)-acetyl] azepan-4ylcarbamoyl}-butylamide}, NPI-8343, NPI-8344, NPI-2349, NPI-2019, NPI-3485 and NPI-3469; and compounds containing alkyl sulfonyls such as described in US Patent 6,030, 946. In a broader sense of the inventi on, the term "cathepsin S inhibitor" as used herein include those compounds disclosed in WO00/49007; WO00/49008; WO00/48992, WO01/30772, WO 97/40066; WO96/40737; WO01/19816; WO01/09110, WO02/20485, WO95/2322, WO96/30353, WO01/19808, WO01/19796, WO00/55144, WO00/55124, WO00/55125, WO00/55126, WO02/51983, WO01/89451, WO00/69855, WO02/40462, WO01/49288, WO02/069901, WO02/070517, WO01/09169, WO00/759881, WO02/20002, WO02/20011, WO02/20012, WO02/20013, WO02/14314, WO02/14315, WO02/14317 and WO00/51998 as well as modulators inhibiting the enzyme activity of cathepsin S that can be found be means of the assays and techniques disclosed in WO03/020287.

One particularly useful class of compounds of cathepsin S inhibitors is the class of 6-aryl-7H-pyrrolo-(2,3-d)-pyrimidine-2-carbonitrile cathepsin inhibitors which can be more generally referred to as N-heteroaryl-carbonitrile cathepsin inhibitors, in particular those disclosed or generically covered by the claims of WO03/020721 (Novartis AG). One particularly useful compound of this class is a compound of formula I (7-(2,2-dimethyl-propyl)-6-thiophen-2-ylmethyl-7.H.-pyrrolo[2,3-.d.]pyrimidine-2-carbonitrile).

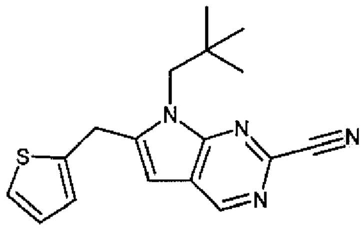

(I)

The compound of formula I is further disclosed in the patent application WO03/020721 and may be synthesized as described therein.

Another particularly useful compound of this class is a compound of formula II and pharmaceutically acceptable salts thereof.

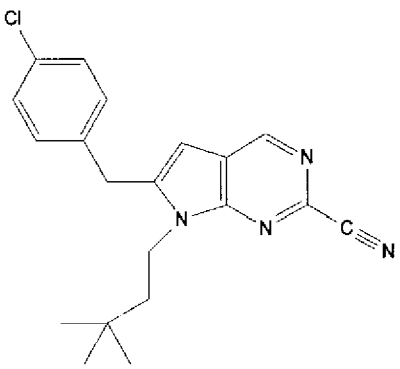

(II)

The compound of formula II can be prepared em ploying the procedures disclosed in the patent application WO03/020721.

In addition, another particularly useful compound is a compound of formula III, 3-[(4-morpholinylcarbonyl)-phenylalanylamido]-1-fluoro-5-phenyl-2-pentanone (disclosed in J. Clin. Invest 1993 Mar 91(3):1052-6 and US Patent 4,518,528).

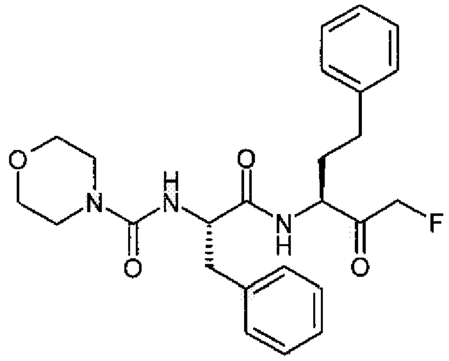

(III)

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

The term "a combined preparation", as us ed herein defines especially a "kit of parts" in the sense that the first and second active ingredient as defined above can be dosed independently or by use of different fixed combinations with distinguished amounts of the ingredients, i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the active ingredients. The ratio of the total amounts of the active ingredient 1 to the active ingredient 2 to be administered in the combined preparation can be varied, e.g., in order to cope with the needs of a patient sub -population to be treated or the needs of the single patient which different needs can be due to age, sex, body weight, etc. of the patients. Preferably, there is at least one beneficial effect, e.g., a mutual enhancing of the effect of the first and second active ingredient, in particular a synergism, e.g. a more than additive effect, additional advantageous effects, less side effects, a combined therapeutical effect in a non-effective dosage of one or both of the first and second active ingredient, and especially a strong synergism the first and second active ingredient.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an add itionally present basic center. The active ingredients having an acid group (for example COOH) can also form salts with bases. The active ingredient or a pharmaceutically acceptable salt thereof may also be used in form of a hydrate or include other solvents used for crystallization.

Brief Discussion of Figure 1

Fig. 1 demonstrates the synergism between a cathepsin S inhibitor and morphine as determined in Example 1. The employed abbreviations have the following meanings: Veh means vehicle, Mor means morphine, and Inhibitor denotes a cathepsin S inhibitor of formula II. The shown value constitutes the paw withdrawal threshold [g].

A combination which comprises a cathepsin S inhibitor and an opioid, in which the active ingredients are present in each case in free form or in the form of a pharmaceutically acceptable salt will be referred to hereinafter as a COMBINATION OF THE INVENTION.

Surprisingly it was found that the adm inistration of a COMBINATION OF THE INVENTION results in a beneficial, especially a synergistic, therapeutic effect or in other surprising beneficial effects, e.g. less side effects, compared to a monotherapy applying only one of the pharmaceutically active ingredients used in the COMBINATION OF THE INVENTION.

In particular, it has been found that a combination which comprises subeffective doses of a catheps in S inhibitor and an opiate achieves the same effect as effective doses of either compound alone.

A further benefit is that lower doses of the active ingredients of the COMBINATION OF THE INVENTION can be used, for example, that the dosages need not only often be smaller but are also applied less frequently, or can be used in order to diminish the incidence of side effects. This is in accordance with the desires and requirements of the patients to be treated.

The pharmacological activity of a COMBINATION OF THE INVENTION for the treatment of pain may, for example, be evidenced in preclinical studies known as such, e.g. the methods described in the Examples.

The pharmacological activity of a COMBINATION OF THE INVENTION may, for example, also be demonstrated in clinical studies. Such clinical studies are preferably randomized, double-blind, clinical studies in patients with chronic pain, e.g. post herpatic neuralgia, diabetic neuropathy and cancer patients. Such studies demonstrate, in particular, the synergism of the active ingredients of the COMBINATION OF THE INVENTION. The beneficial effects on pain can be determined directly through the results of these studies or by changes in the study design which are known as such to a person skilled in the art. The studies are, in particular, suitable to compare the effects of a monotherapy using the active ingredients and a COMBINATION OF THE INVENTION.

It is one objective of this invention to provide a pharmaceutical composition comprising a quantity, which is jointly therapeutically effective against pain, comprising the COMBINATION OF THE INVENTION and at least one pharmaceutically acceptable carrier. In this composition, the first and second active ingredient can be administered together, one after the other or separately in one com bined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active ingredient, alone or in combination with one or more pharmaceutically acceptable carrier, especially suitable for enteral or parenteral appl ication. The preferred routes of administration of the dosage forms of the present invention are orally, intravenously or nasally.

The novel pharmaceutical composition contain, for example, from about 10 % to about 100 %, preferably from about 20 % to about 60 %, of the active ingredients. Pharmaceutical preparations for the combination therapy for enteral or parenteral adm inistration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of active ingredient or ingredients contained in an individual dose of each dosage form need not in itself constitute an effective amount since the necessary effective amount can be reached by administration of a plurality of dosage units.

In preparing the com positions for oral dosage form, any of the usual pharmaceutical media may be employed, such as, for example, water, glycols, oils or alcohols; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dos age unit form in which case solid pharmaceutical carriers are obviously employed.

In particular, the present invention relates to a pharmaceutical composition suitable for synergistic action of the active components against pain which comprises a synergistically effective amount of a COMBINATION OF THE INVENTION and at least one pharmaceutically acceptable carrier.

Furthermore, the present invention relates to the use of a COMBINATION OF THE INVENTION for the preparation of a medicament for the treatment of pain, especially chronic pain.

Additionally, the present invention provides a method of treating or ameliorating pain, especially chronic pain, in a warm-blooded animal in need thereof comprising administering to the animal a COMBINATION OF THE INVENTION in a quantity which is jointly therapeutically effective against pain and in which the compounds can also be present in the form of their pharmaceutically acceptable salts.

Moreover, the present invention provides a commercial package comprising as active ingredients COMBINATION OF THE INVENTION, together with instructions for simultaneous, separate or sequential use thereof in the treatment of pain.

In particular, a therapeutically effective amount of each of the active ingredients of the COMBINATION OF THE INVENTION may be administered simultaneously or sequentially and in any order, and the components may be administered separately or as a fixed combination. For example, the method of treatment of diseases according to the invention may comprise (i) administration of the first active ingredient in free or pharmaceutically acceptable salt form and (ii) administration of the second active ingredient in free or pharmaceutically acceptable salt form, simultaneously or sequentially in any order, in jointly therapeutically effective amounts, preferably in synergistically effective amounts, e.g. in daily dosages corresponding to the amounts described herein. The individual active ingredients of the COMBINATION OF THE INVENTION can be administered separately at different times during the course of therapy or concurrently in divided or single combination forms. Furthermore, the term administering also encompasses the use of a prodrug of an active ingredient that convert *in vivo* to the active ingredient. The instant invention is therefore to be understood as embracing all such regimes of simultaneous or alternating treatment and the term "administering" is to be interpreted accordingly.

The effective dosage of each of the active ingredients em ployed in the COM BINATION OF THE INVENTION may vary depending on the particular compound or pharmaceutical composition employed, the mode of administration, the severity of the condition being treated. Thus, the dosage regim en the COM BINATION OF THE INVENTION is selected in accordance with a variety of factors including the route of administration and the renal and hepatic function of the patient. A physician, clinician or veterinari an of ordinary skill can readily determine and prescribe the effective amount of the single active ingredients required to prevent, ameliorate or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients. The dose of an opioid generally will be between 75 ng and 750 mg, depending on the opioid chosen.

When the combination partners employed in the COMBINATION OF THE INVENTION are applied in the form as marketed as single drugs for the indication pain, their dosage and mode of administration can take place in accordance with the information provided on the packet leaflet of the respective marketed drug in order to result in the beneficial effect described herein, if not mentioned herein otherwise.

The following Examples serve to illustrate the invention without limiting the invention in its scope.

<u>Example 1: Chronic neuropathic pain model</u>

Hyperalgesia is examined in the model of neuropathic pain induced by partial ligation of the sciatic nerve as described by Seltzer et al. (1990). Briefly, Wistar rats (120-140 g) are anaesthetised, the left sciatic nerve exposed at mid-thigh level through a small incision and 1/3 to 1/2 of the nerve thickness tightly ligated within a 7.0 silk suture. The wound is closed with a single muscle suture and skin clips and dusted with Aureomycin antibiotic powder. The animals are allowed to recover and used 12-15 days following surgery.

Mechanical hyperalgesia is assessed by measuring paw withdrawal thresholds to an increasing pressure stimulus placed onto the dorsal surface of the paw using an analgesymeter (Ugo-Basile, Milan) with a cut-off of 250 g. Withdrawal thresholds are measured on both the ipsilateral (ligated) and contralateral (unligated) paw prior to (predose) and then up to 6 h following drug or vehicle administration. Data are expressed as withdrawal threshold (g) and percentage reversal of hyperalgesia calculated according to the following formula:

$$\%\ reversal = \frac{ipsilateral\ threshold\ postdose - ipsilateral\ threshold\ predose}{contralateral\ threshold\ predose - ipsilateral\ threshold\ predose}\ X\ 100$$

Potency is expressed as $D_{50}$ value, i.e. the dose of the single compound or a COMBINATION OF THE INVENTION necessary to produce 50 % reversal of hyperalgesia.

Paw withdrawal thresholds are measured prior to drug treatment (predose) and then at 0.5, 1, 3 and 6 hou rs following concomitant administration of morphine subcutaneously and the cathepsin inhibitor of formula II orally. Each time point represents data from 6 animals per group. Vehicle is 0.5% methylcellulose, 1 ml p.o./0.9% saline s.c. Statistical analysis is performed on raw data (paw withdrawal thresholds).

Statistical significance, *† P<0.05 by Analysis of variance followed by Dunnett's test.

<u>Table 1</u>: Effect of concomitant administration of the cathepsin inhibitor of formula II ("Inhibitor") with morphine on established mechanical hyperalgesia in the Seltzer model of chronic neuropathic pain

| % Reversal of Predose Hyperalgesia | | | | | |
|---|---|---|---|---|---|
| Time post-dose (h) | Vehicle | Morphine 1mg/kg | Inhibitor 10 mg/kg | Morphine+ Inhibitor | Inhibitor 30 mg/kg |
| 0.5<br>1 | 8.6±2.7<br>8.9±4.6 | 4.4±2.8<br>6.5±2.9 | 30.3±5.8<br>22.8±4.6 | 55.8±9.2*<br>51.6±9.0*† | 46.7±11.4<br>54.7±7.1 |
| Values are mean ± s.e.m. * p < 0.05 morphine + Inhibitor *versus* morphine, † p < 0.05 morphine + Inhibitor *vs* Inhibitor {10 mg/kg). Morphine (1 mg/kg) and Inhibitor (10 mg/kg) does not achieve statistically significant effect at any time point. Inhibitor (30 mg/kg) reaches significant effect at 1 point vs control vehicle group. | | | | | |

The data ind icate that in the Seltzer model of neuropathic pain, a sub-effective dose of the cathepsin inhibitor

of formula II (10 mg/kg p.o.) administered concomitantly to a sub-effective dose of morphine (1 mg/kg s.c.) reverses established mechanical hyperalgesia to the same extent as an effective dose of the cathepsin inhibitor of formula II (30 mg/kg) (Table 1).

This data indicates that an interaction occurs between the two drugs that are I ikely to exhibit different mechanisms of action and shows that Cathepsin S inhibitors can potentiate the anti-hyperalgesic effect of systemic morphine.

Example 2.

Naloxone hydrochloride blocks the anti-hyperalgesic effect in neuropathic rats induced by the compound of formula II. The dose of naloxone used (0.1 mg/kg) is devoid of any effect on neuropathic rat nociceptive threshold. This data suggests that the effect of Cathepsin S inhibitors is mediated by the opioid system.

## Claims

1. A combination comprising a cathepsin S inhibitor and an opioid, in which the active ingredients are present in each case in free form or in the form of a pharmaceutically acceptable salt and optionally at least one pharmaceutically acceptable carrier; for simultaneous, separate or sequential use.

2. Combination according to claim 1 which is a combined preparation or a pharmaceutical composition.

3. Combination according to claim 1 or 2 comprising an opioid selected from the group consisting of alfentanil, allylprodine, alphaprodine, anileridine, benzylmorphine, bezitramide, buprenorphine, butorphanol, clonitazene, codeine, cyclorphan, desomorphine, dextromoramide, dezocine, diampromide, dihydrocodeine, dihydromorphine, eptazocine, ethylmorphine, fentanyl, hydrocodone, hydromorphone, hydroxypethidine, levophenacylmorphan, levorphanol, lofentanil, methylmorphine, morphine, necomorphine, normethadone, normorphine, opium, oxycodone, oxymorphone, pholcodine, profadol and sufentanil.

4. Combination according to any one of claims 1 to 3 comprising a 6-aryl-7H-pyrrolo-(2,3-d)-pyrimidine-2-carbonitrile disclosed or generically covered by the claims of WO03/020721.

5. Combination according to any one of claims 1 to 3 comprising a compound of formula II or

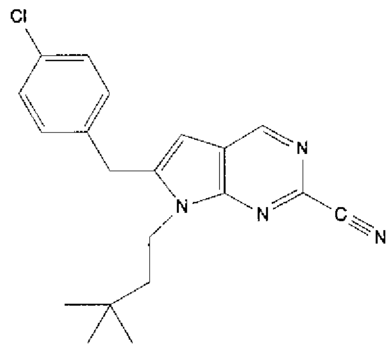

(II)

a pharmaceutically acceptable salt thereof.

6. Combination according to any one of claims 1 to 5 for simultaneous, separate or sequential use in the treatment of pain.

7. Combination according to claim 6 wherein the cathepsin S inhibitor and the opioid produce a synergistic therapeutic effect.

8. Method of treating or ameliorating pain in a warm-blooded animal in need thereof comprising administering to the animal a combination according to any one of claims 1 to 5 in a quantity which is jointly therapeutically effective

against pain and in which the compounds can also be present in the form of their pharmaceutically acceptable salts.

**9.** A pharmaceutical composition comprising a quantity, which is jointly therapeutically effective against pain, of a combination according to any one of claims 1 to 5 and at least one pharmaceutically acceptable carrier.

**10.** Use of a combination according to any one of claims 1 to 5 for the preparation of a medicament for the treatment of pain.

**11.** Use according to claim 6 or 10 or method according to claim 8 wherein the pa in is chronic pain.

**12.** A commercial package comprising a combination according to any one of claim 1 to 5 together with instructions for simultaneous, separate or sequential use thereof in the treatment of pain, especially chronic pain.

Fig. 1

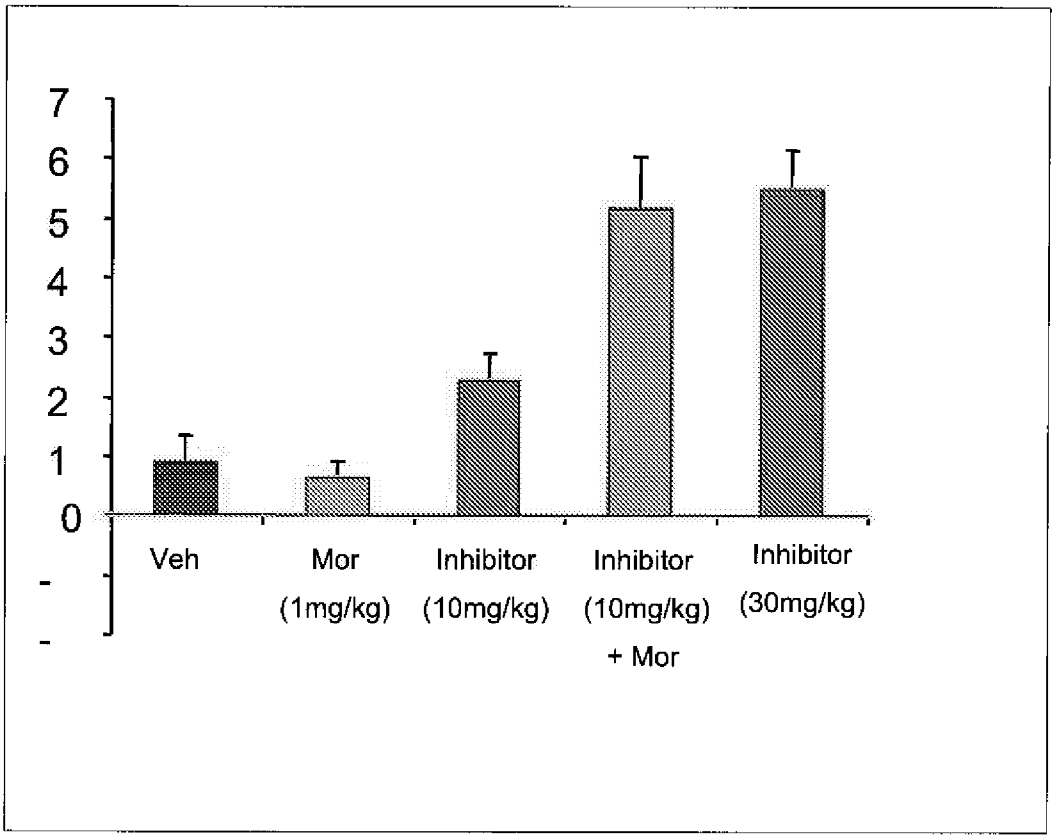
7
6
5
4
3
2
1
0
-
-
Veh
Mor
(1mg/kg)
Inhibitor
(10mg/kg)
Inhibitor
(10mg/kg)
+ Mor
Inhibitor
(30mg/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03020287 A
- WO 9924460 A
- US 4518528 A
- US 5374623 A
- US 5486623 A
- US 6030946 A
- WO 0049007 A
- WO 0049008 A
- WO 0048992 A
- WO 0130772 A
- WO 9740066 A
- WO 9640737 A
- WO 0119816 A
- WO 0109110 A
- WO 0220485 A
- WO 952322 A
- WO 9630353 A
- WO 0119808 A
- WO 0119796 A
- WO 0055144 A
- WO 0055124 A
- WO 0055125 A
- WO 0055126 A
- WO 0251983 A
- WO 0189451 A
- WO 0069855 A
- WO 0240462 A
- WO 0149288 A
- WO 02069901 A
- WO 02070517 A
- WO 0109169 A
- WO 00759881 A
- WO 0220002 A
- WO 0220011 A
- WO 0220012 A
- WO 0220013 A
- WO 0214314 A
- WO 0214315 A
- WO 0214317 A
- WO 0051998 A
- WO 03020721 A


### Non-patent literature cited in the description


- *J. Clin. Invest,* 09 March 1993, vol. 1 (3), 1052-6